# EUROPEAN PATENT SPECIFICATION

(11) **EP 4 091 570 B1**
(45) Date of publication and mention of the grant of the patent: **14.02.2024**
(21) Application number: 22174287.7
(22) Date of filing: 19.05.2022
(51) Int. Cl.: A61B 34/20

(54) **PROBE FOR IMPROVING REGISTRATION ACCURACY BETWEEN A TOMOGRAPHIC IMAGE AND A TRACKING SYSTEM**
SONDE ZUM VERBESSERN DER AUSRICHTUNGSGENAUIGKEIT ZWISCHEN EINEM TOMOGRAFISCHEN BILD UND EINEM VERFOLGUNGSSYSTEM
SONDE PERMETTANT D'AMÉLIORER LA PRÉCISION D'ENREGISTREMENT ENTRE UNE IMAGE TOMOGRAPHIQUE ET UN SYSTÈME DE SUIVI

(30) Priority: 20.05.2021 US 202117325353
(43) Date of publication of application: 23.11.2022
(73) Proprietor: Biosense Webster (Israel) Ltd., Yokneam, 2066717 (IL)
(72) Inventor: SEGAL, Iris, 2066717 Yokneam (IL); WOLFSON, Helen, 2066717 Yokneam (IL); GUSEIN, George, 2066717 Yokneam (IL); HOD, Uriel, 2066717 Yokneam (IL); PELED, Ran, 2066717 Yokneam (IL)
(74) Representative: Carpmaels & Ransford LLP

(56) References cited:
- CN-A- 105 559 885
- US-A1- 2010 063 387
- US-A1- 2018 071 031

## Description

### FIELD OF THE INVENTION

The present invention relates generally to medical imaging and mapping, and specifically to registration of medical images with a position tracking system.

### BACKGROUND

In image-guided surgery a medical practitioner uses instruments that are tracked in real time so that positions and/or orientations of the instruments may be presented on images of a patient's anatomy during a surgical procedure. In some cases both the tracking and the imaging of the patient's anatomy may be implemented by one modality, such as fluoroscopy. However, because fluoroscopy uses ionizing radiation, its use should be minimized. Consequently in many scenarios an image of the patient is prepared in one modality, such as magnetic resonance imaging (MRI) or computerized tomography (CT) fluoroscopy, and the instrument tracking uses a different modality, such as magnetic tracking.

For accurate utilization of imaging in conjunction with instrument tracking, the two modalities should be accurately registered with each other. For this purpose, for example, United States Patent Application Publication 2019/0046272, whose disclosure is incorporated herein by reference, describes a method, which includes receiving a CT image of voxels of a subject's head, and analyzing the image to identify respective locations of the subject's eyes in the image, so defining a first line segment joining the respective locations. The method includes identifying a voxel subset overlaying bony sections of the head, lying on a second line segment parallel to the first line segment and on a third line segment orthogonal to the first line segment. A magnetic tracking system configured to measure positions on the subject's head is activated, and a probe, operative in the system, is positioned in proximity to the bony sections to measure positions of a surface of the head overlaying the bony sections. A correspondence between the positions and the voxel subset is formed, and a registration between the CT image and the magnetic tracking system is generated in response to the correspondence.

As another example, United States Patent 10,265,137 describes a template, which may be aligned to the patient and instruments passed though the guide elements and into various targets. The template may be aligned using one or more of, for example, a position sensing system or a live imaging modality to register the patient to the template.

United States Patent Application Publication 2019/0282301 describes image guidance software comprising an algorithm for registering a virtual device model to the image space automatically, approximating the most likely configuration of a device on the skin surface before the actual device is placed on the patient. The virtual model of the device and its reachable area are overlaid on the planning image data, in order to visualize and quantify the reachability of the target.

United States Patent Application Publication 2006/0116576 describes systems and method for navigating a medical probe (such as a catheter) relative to an anatomical body (such as a heart). A mark (such as a point or line), representing an anatomical region of interest (such as tissue targeted for treatment or tissue not targeted for treatment) is displayed on a representation of the anatomical body. The positions of the medical probe and the mark are determined within a three-dimensional coordinate system, and the proximity between the medical probe and the mark determined based on these positions. This proximity can then be indicated to a user, e.g., using graphics, text, or audible sounds.

United States Patent 8,657,809 describes a surgical navigation system, which includes a camera that is fixedly attached to a patient. The camera space merges with the patient space and thereby eliminates the need for a separate patient tracker. The surgical navigation system calculates the position of a surgical tool with a tracking device in view of the camera and shows on a display device the position of the surgical tool with respect to the patient superimposed and in correlation with a scan image of the patient.

United States Patent Application Publication 2014/0343408 describes an apparatus and methods for use with an imaging device that acquires an external image of a multi-lumen structure within a subject's body. A tool with a location sensor coupled thereto is moved along branches of the structure. The multi-lumen structure is registered with the external image, such that the multi-lumen structure and the external image of the multi-lumen structure have a common frame of coordinates, by sensing the location coordinates of the tool while the tool moves along a plurality of branch lines within the multi-lumen structure, the branch lines defining a plurality of paths within the multi-lumen structure. The branch lines are identified within the image, and a correspondence between the sensed location coordinates of the tool and the plurality of branch lines within the image is determined.

US2018/071031A1 describes a system including a data collection unit and a registration probe. The registration probe comprises a registration probe body that can have a distal portion, intermediate portion, a proximal portion, and a longitudinal axis defined by the intermediate portion. The registration probe can further comprise a dynamic engagement tip operably coupled to a distal end of the distal portion. The dynamic engagement tip can be rotatable with respect to the registration probe. The registration probe can also comprise a locator coupled to the proximal portion. In operation, the dynamic engagement tip of the registration probe can be placed in continuous contact with and moved around a selected anatomical area during a selected time period where the data collection system collects physical space data corresponding to individual surface points at a data collection rate. The data collection unit can determine at least one of a plurality of point-based registrations and distance between individual surface points based on the collected physical space data.

### SUMMARY

The invention is disclosed in the appended claims.

There is therefore provided a medical apparatus including a probe, which includes a handle having a longitudinal axis, a distal tip disposed on the longitudinal axis, and a position sensor, which is disposed in the handle on the longitudinal axis at a predefined distance from the distal tip and is configured to output a signal indicative of a location of the probe. An alignment jig includes a connector configured to be fixed removably to the distal tip of the probe and three protrusions, which extend from the connector and are configured to contact a surface of a body of a patient at respective points, which are disposed in a plane perpendicular to the longitudinal axis when the connector is fixed to the distal tip of the probe.

In a disclosed embodiment, the connector includes a socket, which fits over the distal tip. Additionally or alternatively, the protrusions include rollers, which are configured to contact and roll over the surface of the body.

There is also provided, in accordance with an embodiment of the invention, a medical apparatus, including a registration probe, which has a longitudinal axis and a distal tip disposed on the longitudinal axis, and which includes an alignment jig including three protrusions, which extend from the distal tip and are configured to contact a surface of a part of a body of a patient at respective points, which are disposed in a plane perpendicular to the longitudinal axis. A position sensor is disposed on the longitudinal axis at a predefined distance from the distal tip. a position-tracking system is configured to acquire position coordinates of the position sensor in a first frame of reference defined by the position-tracking system in a vicinity of the part of the body of the patient. A processing unit is configured to receive three-dimensional (3D) image data with respect to at least the part of the body of the patient in a second frame of reference, to receive the position coordinates acquired by the position-tracking system while the distal tip of the registration probe contacts the locations on the surface of the part of the body of the patient, and to register the first and second frames of reference by comparing the position coordinates to corresponding locations in the three-dimensional image data.

In a disclosed embodiment, the position-tracking system includes a magnetic position-tracking system. Additionally or alternatively, the three-dimensional image data includes data from a computerized tomography (CT) system or from a magnetic resonance imaging (MRI) system. In one embodiment, the part of the body of the patient includes a head of the patient, and the 2D image shows a face of the patient.

In some embodiments, registering the first and second frames of reference includes applying relative scaling, rotations and translations between the first and second frames of reference so as to maximize a correlation between the position coordinates and the corresponding locations in the three-dimensional image data.

Additionally or alternatively, the processing unit receives the landmark locations from a practitioner operating the registration probe to mark the locations on the 2D image.

There is additionally provided, in accordance with an embodiment of the invention, a method for registering medical images. The method includes providing a probe including a handle having a longitudinal axis, a distal tip disposed on the longitudinal axis, and a position sensor, which is disposed in the handle on the longitudinal axis at a predefined distance from the distal tip and is configured to output a signal indicative of a location of the probe. An alignment jig is fitted over the distal tip of the probe, wherein the alignment jig includes three protrusions, which extend from the connector and are configured to contact a surface of a body of a patient at respective points, which are disposed in a plane perpendicular to the longitudinal axis when the connector is fixed to the distal tip of the probe. The probe is applied in registering a position-tracking system, which is configured to acquire position coordinates of the position sensor, with a frame of reference of three-dimensional (3D) image data with respect to at least a part of the body of the patient.

In a disclosed embodiment, the alignment jig is fitted removably over the distal tip.

Additionally or alternatively, the part of the body of the patient includes a head of the patient, and applying the probe includes bringing the alignment jig into contact with a face of the patient.

The present invention will be more fully understood from the following detailed description of the embodiments thereof, taken together with the drawings in which:

### BRIEF DESCRIPTION OF THE DRAWINGS

Fig. 1 is a schematic pictorial illustration of a medical apparatus, in accordance with an embodiment of the invention;
Fig. 2 is a schematic pictorial illustration of a registration probe with an alignment jig contacting a patient, in accordance with an embodiment of the invention;
Figs. 3a and 3b are schematic detail views of the registration probe and alignment jig of Fig. 2 separated from and fixed to each other, respectively, in accordance with an embodiment of the invention; and
Figs. 4a and 4b are schematic side and frontal views, respectively, of the probe and alignment jig of Fig. 2, in accordance with an embodiment of the invention.

### DETAILED DESCRIPTION OF EMBODIMENTS

Magnetic tracking systems are used for tracking instruments in invasive diagnostic and therapeutic procedures, such as image-guided surgery, using pre-acquired images, such as CT images, of the part of the body of the patient undergoing the procedure. In order for the tracking to be effective, frames of reference of the image and the tracking system have to be registered with each other. In a typical registration procedure between, for example, a CT image and a magnetic tracking system, the coordinates of a number of different anatomical points (referred to as landmarks) are marked in the CT image, and the coordinates of the same landmarks are acquired by the tracking system. Once pairs of such coordinate points have been acquired, a fitting process is applied in order to estimate the transformation, including scaling, rotation and translation, that best aligns, i.e., registers, the two sets of points. The fit may be computed, for example, using algorithms that are known in the art, such as cumulative distance metric or an iterative closest point (ICP) algorithm.

As an example, we will consider a procedure requiring tracking of an instrument used on a patient's head, such as an ear, nose, and throat (ENT) procedure. In such a procedure, the head of the patient is registered with the frame of reference of the tracking system. Example of such a registration procedure is described in United States Patent Application 16/704,042, filed December 5, 2019.

In the described procedure, a processing unit of a magnetic tracking system receives 3D image data of a head of the patient, and generates a 2D image of the surface of the part of the head based on the 3D image data. The processing unit further renders the 2D image to a display screen, and superimposes, under guidance from a medical practitioner, such as a physician, onto the displayed 2D image icons indicating locations of respective landmarks on the surface of the head. The physician touches with the distal end of a registration probe comprising a magnetic position sensor, guided by these icons on the 2D image, the corresponding points on the patient's head and indicates to the processing unit which point he/she has touched. The processing unit receives the corresponding 3D position coordinates of the position sensor acquired by the position-tracking system from these points. Finally, the processing unit registers the frame of reference of the position-tracking system with the frame of reference of the 3D image data by relative translations and rotations of the two frames of reference, until the correlation between the 3D coordinates acquired by the position-tracking system and the coordinates of the 3D image data corresponding to the icons is maximized.

Errors may, however, be introduced into the recorded 3D coordinates of the patient's head and cause a concomitant error in the registration between the frames of reference of the image and the tracking system. One type of error is caused by the registration probe being held so that its longitudinal axis (the axis connecting the distal end and the position sensor) is not perpendicular to the patient's skin. Even when the position sensor measures the angular orientation of the probe, in addition to the 3D location coordinates of the sensor, significant errors can still arise in maintaining the desired perpendicular orientation of the probe relative to the surface of the patient's head (i.e., the skin). This error, in turn, leads to an inaccuracy in the estimated location of the distal end of the registration probe.

Another error may be caused by the distal end of the registration probe indenting the skin due to pressure exerted by the practitioner. This indentation can be particularly significant in soft areas of the head (for example, cheeks) and for patients whose skin is swollen, for example due to medication. Yet another error may be caused by the registration probe dragging the skin sideways, which can occur in elderly patients with loose skin.

The embodiments of the present invention that are described herein address this problem by providing an alignment jig at the distal end of the registration probe, to ensure that the probe is held perpendicular to the skin surface and indents the skin only minimally. To enhance sterility, the jig can advantageously be produced as a disposable plastic part, which fits over the distal tip of the registration probe; although alternatively, the jig may be a fixed, integral part of the probe. The jig comprises three protrusions, which extend distally from the probe and contact the surface of the patient's body at respective points, which lie in a plane perpendicular to the longitudinal axis of the probe. The protrusions divide the pressure exerted by the practitioner and thus assist the practitioner in holding the probe perpendicular to the body surface without indenting the skin too deeply. When the probe is to be used in registering a tracking system with an image of the patient's face, the jig is made sufficiently narrow to reliably contact all areas of the face, such as the nose. Additionally or alternatively, the protrusions comprise rollers, which aid in shifting the probe smoothly across the patient's skin.

In the disclosed embodiments, a medical apparatus includes a registration probe comprising a handle having a longitudinal axis, with a distal tip on the longitudinal axis. A position sensor is mounted in the handle of the registration probe on the longitudinal axis, at a certain predefined distance from the distal tip. The position sensor outputs a signal indicative of the location of the registration probe. An alignment jig comprises a connector, which is fixed removably to the tip of the registration probe. Three protrusions extend from the connector and contact the surface of a patient's body at respective points. As noted earlier, these points are disposed in a plane that is perpendicular to the longitudinal axis of the handle when the connector is fixed to the tip of the registration probe.

The three-point contact of the jig establishes the angular orientation of the registration probe with respect to the skin with greater accuracy and ease than can be accomplished with a single-point registration probe. Thus, the jig ensures accurate and repeatable perpendicular orientation of the probe with respect to the skin while reducing the possible indentation of the skin by the distal end of the probe. These features of the alignment jig improve the accuracy and repeatability of the recorded position of the distal tip of the registration probe on the skin of the patient, thus improving the registration between the frames of reference of the image and the tracking system.

### SYSTEM DESCRIPTION

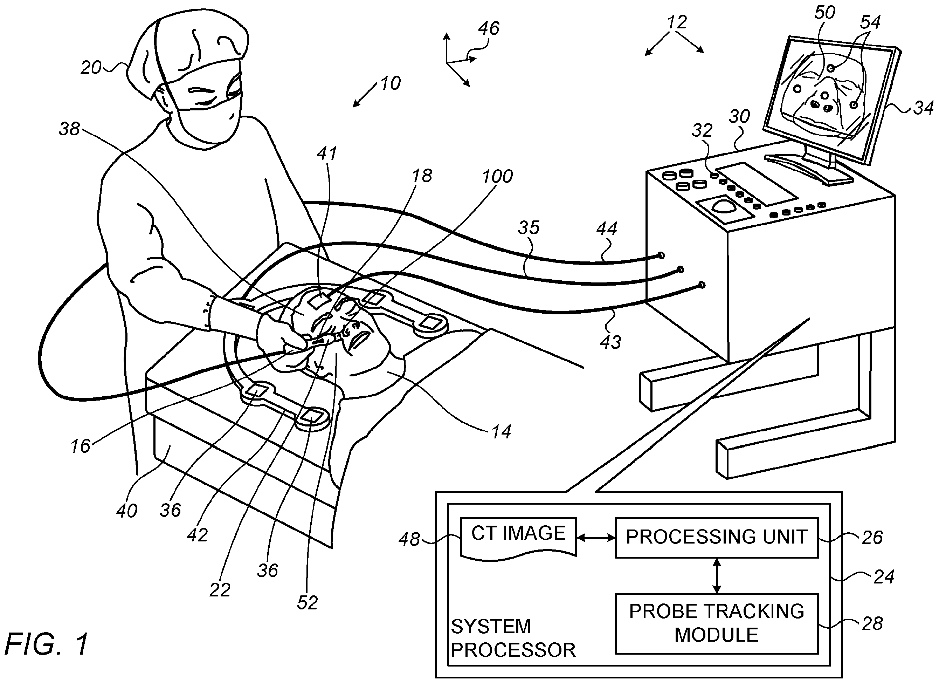

Fig. 1 is a schematic pictorial illustration of a medical apparatus 10, in accordance with an embodiment of the invention. As described in United States Patent Application 16/704,042, referenced hereinabove, apparatus 10 is used to register a magnetic position-tracking system 12 with an image, which is assumed in the present example to comprise a CT image 48, of a patient 14. Position-tracking system 12 in the present example comprises a magnetic tracking system. The TruDi^{®} navigation system, produced by Acclarent, Inc. (Irvine, California), uses a tracking system similar to that described herein to track the location and orientation of the distal tip of a probe inserted into or brought into the vicinity of a patient.

Position-tracking system 12 is used to track positions and orientations of one or more instruments, such as catheters or guidewires, that are inserted into patient 14 during a medical procedure performed on the patient. As is described below, position-tracking system 12 is also able to track the position, and possibly also the orientation, of a registration probe 16 that is external to the patient. Probe 16 comprises a handle 18 that may be held by a medical practitioner 20, typically a physician, during use of system 10 for positioning the probe in a desired location and orientation. Probe 16 further comprises a distal tip with an alignment jig 100 and a position sensor 22, whose position and orientation are tracked by position tracking system 12.

For clarity and simplicity in the following description, the medical procedure referred to above is assumed to comprise an invasive procedure on a nasal sinus of patient 14, so that medical apparatus 10 and magnetic position-tracking system 12 are assumed to be configured to operate in and around the region of the nasal sinus. However, systems 10 and 12 may alternatively be configured to operate in and around other regions of a patient, such as the thorax, kidneys or abdomen, and those having ordinary skill in the art will be able to adapt the elements of the systems that are described herein for operation in such other regions. Furthermore, the principles of the present invention may be applied in conjunction with other types of tracking systems (not necessarily magnetic), as well as other sorts of 3D imaging modalities, such as MRI.

Tracking system 12 is operated by a system processor 24, comprising a processing unit 26 communicating with a probe tracking module 28. The function of module 28 is described below. System processor 24 may be mounted in a console 30, which comprises operating controls 32 that typically include a pointing device such as a mouse or trackball. Physician 20 uses operating controls 32 to transmit commands to system processor 24, which, as described below, is further used to present to the physician data and guiding imagery on a display screen 34.

System processor 24 typically comprises a programmable processor, which uses software stored in a memory of processing unit 26 to operate apparatus 10. The software may be downloaded to system processor 24 in electronic form, over a network, for example, or it may, alternatively or additionally, be provided and/or stored on non-transitory tangible media, such as magnetic, optical, or electronic memory. System processor 24 further stores digitized 3D CT image 48 of head 38 of patient 14, wherein the CT image has been acquired by a separate CT system (not shown), possibly at a different point in time. CT image 48 comprises the 3D coordinates of each point in the image, as well as the radiographic density of the image at each point, with the density given in Hounsfield units, for example.

In order to track the instruments referred to above within patient 14, as well as to track probe 16, processing unit 26 uses probe tracking module 28 to operate, via a cable 35, a plurality of magnetic field generators 36, such as coils. In one embodiment, typically applicable if patient 14 is anesthetized and has a recumbent immobile head 38 on a bed 40, generators 36, as illustrated in Fig. 1, are fixed to a frame 42 placed on the bed, beside the patient's head. In an alternative embodiment (not shown), applicable if patient 14 is not anesthetized, generators 36 are fixed with respect to each other and to a frame attached to head 38. A three-axis reference coil 41 is fixed to head 38, and connected to processing unit 26 by a cable 43.

Generators 36 radiate alternating magnetic fields into and around head 38 of patient 14, and these fields generate signals in magnetic detectors in the instruments and in position sensor 22 of probe 16. The signals are conveyed back to processing unit 26 and probe tracking module 28, via a cable 44 connecting probe 16 to console 30. The processing unit and the module together analyze the signals to derive location and orientation coordinates of the instruments and probe 16 with respect to generators 36. Magnetic field generators 36 thus define a coordinate frame of reference 46 of magnetic tracking system 12.

During the process of registration, as detailed in the above-referenced United States Patent Application 16/704,042, processing unit 26 accesses 3D CT image 48 and renders it into a 2D image 50 on display screen 34. Processing unit 26 further superimposes icons 54 onto selected points on 2D image 50, typically corresponding to anatomical landmarks on the patient's face. Physician 20 brings alignment jig 100 of registration probe 16 into contact with a surface 52 of patient 14 (i.e., with the skin of the patient) at each point corresponding to an icon 54 in turn, and processing unit 26 records the 3D coordinates of the probe at each of these points.

Using these recorded coordinates and the coordinates of the corresponding points in the frame of reference of CT image 48, processing unit 26 registers the frame of reference of position-tracking system 12 with the frame of reference of 3D CT image 48 by computing a transformation including relative scaling, rotations and translations of the two frames of reference. Typically, the transformation is found by a fitting process, which maximizes the correlation between the registered 3D coordinates of the probe and the 3D coordinates of CT image 48 corresponding to icons 54. For maximizing the correlation, processing unit 26 may use algorithms such as cumulative distance metric or an iterative closest point (ICP) algorithm. After the initial registration using icons 54, the accuracy of the registration may be increased by superimposing additional icons on image 50 to be contacted by physician 20 using registration probe 16 and to be recorded by processing unit 26.

A communication between physician 20 and processing unit 26 is established in order to indicate which point on surface 52 is touched. For example, processing unit 26 may cause an icon 54 to flash on display screen 34, thus indicating to physician 20 the point he/she is expected to touch. Once physician 20 has touched this point, he/she indicates through controls 32 (for example, by pressing a key in the keypad or clicking the mouse) that the point has been touched. Alternatively, each icon 54 may be numbered using a numerical sequence, and physician 20 indicates through controls 32 which of the icons he/she has touched.

### REGISTRATION PROBE AND JIG

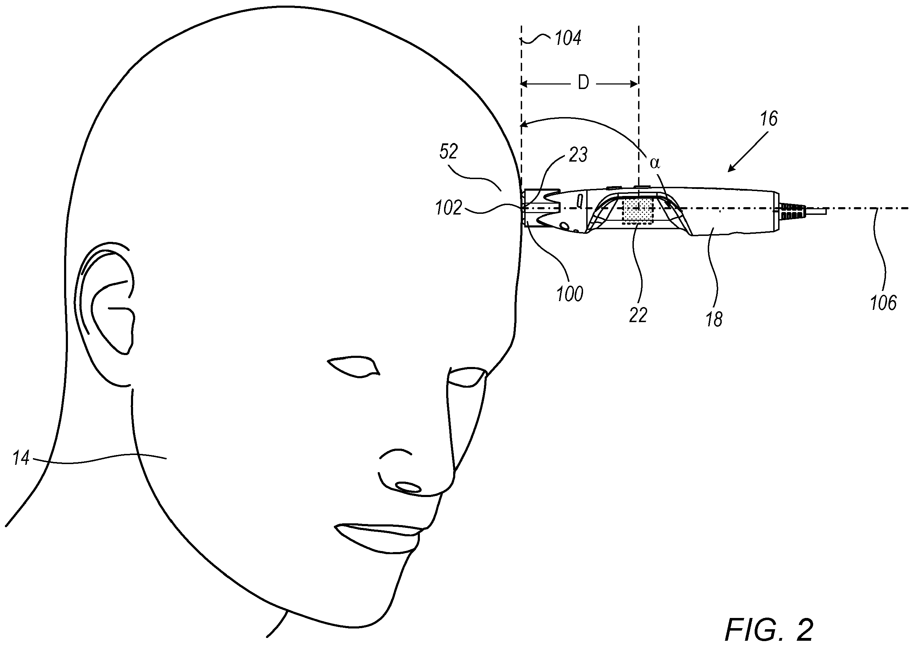

Fig. 2 is a schematic pictorial illustration of registration probe 16 with alignment jig 100 contacting patient 14, in accordance with an embodiment of the invention.

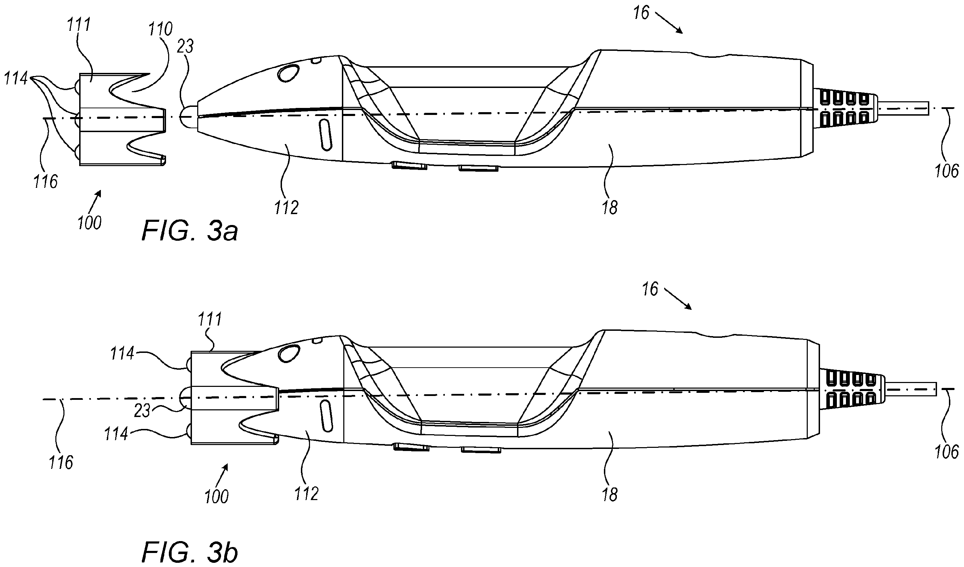

Jig 100 touches surface 52 of patient 14 (i.e., the skin of the patient) at a location 102. A line 104 indicates schematically a plane P that is tangent to surface 52 at location 102. A longitudinal axis 106 of handle 18 forms an angle α with respect to line 104 (plane P). Position sensor 22 in handle 18 is offset from distal tip 23 by a distance D, for example 4 cm. In order to reduce errors of registration due to this offset, it is desirable to hold probe 16 perpendicular to surface 52, i.e., to maintain angle α at 90°. In the pictured embodiment, alignment jig 100 (shown in detail in Figs. 3a and 3b and described hereinbelow) assures perpendicularity of registration probe 16 (and specifically of longitudinal axis 106) to surface 52, i.e., to the skin of patient 14.

Alignment jig 100 is also useful in reducing registration error due to compressing or displacing the skin of patient 14 in a direction perpendicular to the skin (perpendicular to plane P), as well as due to lateral movement of probe 16 along the skin.

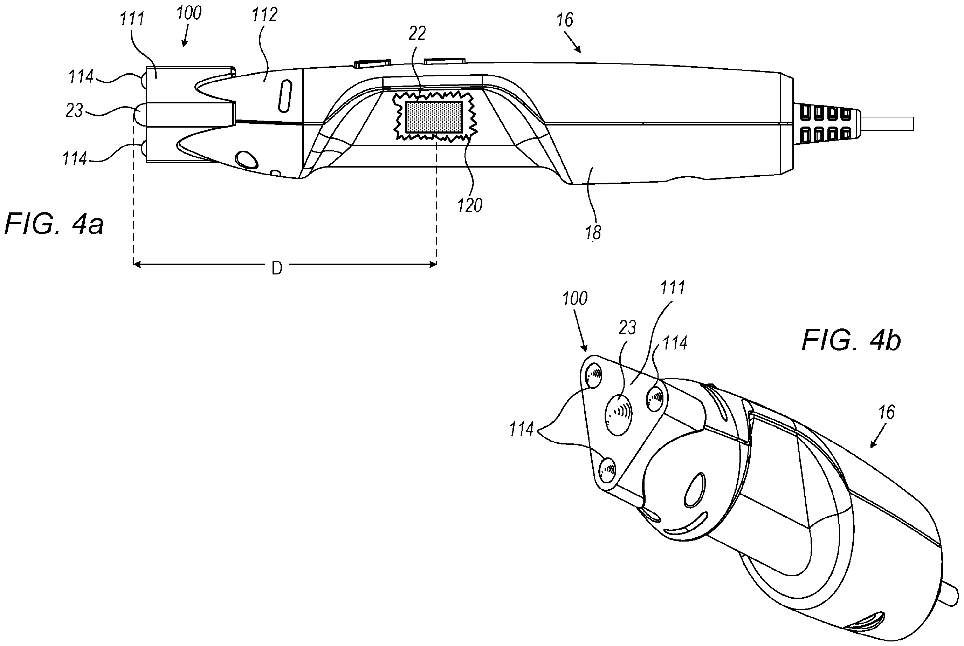

Reference is now made to Figs. 3a, 3b 4a, and 4b, which schematically show details of registration probe 16 and alignment jig 100, in accordance with an embodiment of the invention. Figs. 3a and 3b are schematic side views of registration probe 16 and alignment jig 100 separated from and fixed to each other, respectively. Figs. 4a and 4b are schematic side and frontal views, respectively, of probe 16 and alignment jig 100.

As shown in Fig. 3a, alignment jig 100 comprises a socket 110, which fits firmly over a rounded conical front end 112 of probe 16 and thus brings the alignment jig and the probe into mutual alignment, so that longitudinal axis 106 of probe and an axis 116 of the alignment jig coincide. A central opening in socket 110 allows distal tip 23 to protrude through alignment jig 100 by a small, predetermined distance.

Three protrusions 114 extend distally from a body 111 of alignment jig 100 and define a plane perpendicular to axis 116 of the alignment jig. The triangular arrangement of protrusions 114 is shown in Fig. 4b. In the pictured embodiment, protrusions 114 comprise rollers, which may be spherical or cylindrical; but alternatively, other types of protrusions may be formed on body 111, with or without rollers.

As shown in Fig. 3b, when alignment jig 100 is fixed to registration probe 16, axes 106 and 116 coincide. Thus, longitudinal axis 106 of probe 16 is also perpendicular to the plane defined by protrusions 114. Consequently, when protrusions 114 contact surface 52 of patient 14 (i.e., the patient's skin), axis 106 of probe 16 is perpendicular to the patient's skin (α = 90°). Position sensor 22 within handle 18 (seen in a cutaway 120 in Fig. 4a) is located at distance D from distal tip 23. Because protrusions 114 touch the skin over a much larger area than distal tip 23 alone, the distal tip touches the patient's skin with only minimal compression and minimal displacement in a direction normal to the skin. Moreover, the rollers of protrusions 114 enable probe 16 to be moved sideways with minimal friction, thus reducing the sideways movement of loose skin and the concomitant registration error.

In the pictured embodiment, alignment jig 100 is a separate entity from probe 16. It may be produced inexpensively, for example from molded plastic, and then disposed of after use, thus avoiding the need for cleaning and sterilization.

## Claims

1. A medical apparatus comprising:
a probe (16) comprising:
a handle (18) having a longitudinal axis (106);
a distal tip (23) disposed on the longitudinal axis; and
a position sensor (22), which is disposed in the handle on the longitudinal axis at a predefined distance (D) from the distal tip and is configured to output a signal indicative of a location of the probe; and
an alignment jig (100), which comprises:
a connector (111) configured to be fixed removably to the distal tip of the probe; and
three protrusions (114), which extend from the connector and are configured to contact a surface (52) of a body of a patient (14) at respective points, which are disposed in a plane perpendicular to the longitudinal axis when the connector is fixed to the distal tip of the probe.

2. The apparatus according to claim 1, wherein the connector comprises a socket (110), which fits over the distal tip.

3. The apparatus according to claim 1, wherein the protrusions comprise rollers, which are configured to contact and roll over the surface of the body.

4. The apparatus according to claim 1, wherein the probe is a registration probe, and
wherein the apparatus further comprises:
a processing unit (26) configured:
to receive three-dimensional (3D) image data with respect to at least a part of the body of the patient in a second frame of reference;
to receive position coordinates of the position sensor in a first frame of reference acquired by a position-tracking system, which defines the first frame of reference, while the distal tip of the registration probe contacts the locations on the surface of the part of the body of the patient; and
to register the first and second frames of reference by comparing the position coordinates to corresponding locations in the three-dimensional image data.

5. The apparatus according to claim 4, wherein the three-dimensional image data comprises data from a computerized tomography (CT) system, or from a magnetic resonance imaging (MRI) system.

6. The apparatus according to claim 4, wherein the part of the body of the patient comprises a head of the patient, and the 2D image shows a face of the patient.

7. The apparatus according to claim 4, wherein registering the first and second frames of reference comprises applying relative scaling, rotations and translations between the first and second frames of reference so as to maximize a correlation between the position coordinates and the corresponding locations in the three-dimensional image data.

8. The apparatus according to claim 4, wherein the processing unit is configured to receive the landmark locations from a practitioner operating the registration probe to mark the locations on the 2D image.

9. A method for registering medical images, the method comprising:
providing a probe (16) comprising a handle (18) having a longitudinal axis (106), a distal tip (23) disposed on the longitudinal axis, and a position sensor (22), which is disposed in the handle on the longitudinal axis at a predefined distance (D) from the distal tip and is configured to output a signal indicative of a location of the probe;
fitting over the distal tip of the probe an alignment jig (100) comprising three protrusions (114), which extend from a connector of the alignment jig and are configured to contact a surface (52) of a body (14) of a patient at respective points, which are disposed in a plane perpendicular to the longitudinal axis when the connector is fixed to the distal tip of the probe; and
applying the probe in registering a position-tracking system (12), which is configured to acquire position coordinates of the position sensor, with a frame of reference of three-dimensional (3D) image data with respect to at least a part of the body of the patient.

10. The method according to claim 9, wherein the alignment jig is fitted removably over the distal tip.

11. The method according to claim 9, wherein the protrusions comprise rollers, which are configured to contact and roll over the surface of the body.

12. The apparatus according to claim 4 or the method according to claim 9, wherein the position-tracking system comprises a magnetic position-tracking system, and, optionally, wherein the apparatus further comprises the position-tracking system.

13. The method according to claim 11, wherein the 3D image data comprises computerized tomography (CT) data, or magnetic resonance imaging (MRI) data.

14. The method according to claim 9, wherein the part of the body of the patient comprises a head of the patient, and applying the probe comprises bringing the alignment jig into contact with a face of the patient.

15. The method according to claim 9, wherein registering the position tracking system comprises applying relative scaling, rotations and translations between the position coordinates and the frame of reference so as to maximize a correlation between the position coordinates and corresponding locations in the 3D image data.

## Patentansprüche

1. Medizinische Vorrichtung, umfassend:
eine Sonde (16), umfassend:
einen Griff (18) mit einer Längsachse (106);
eine distale Spitze (23), die auf der Längsachse angeordnet ist; und
einen Positionssensor (22), der in dem Griff auf der Längsachse in einem vordefinierten Abstand (D) zu der distalen Spitze angeordnet ist und konfiguriert ist, um ein Signal auszugeben, das einen Ort der Sonde angibt; und
eine Ausrichtungseinrichtung (100), welche umfasst:
ein Verbindungsstück (111), das ausgestaltet ist, um entfernbar an der distalen Spitze der Sonde fixiert zu werden; und
drei Vorsprünge (114), die sich von dem Verbindungsstück erstrecken und konfiguriert sind, um eine Oberfläche (52) eines Körpers eines Patienten (14) an jeweiligen Punkten zu kontaktieren, die in einer Ebene senkrecht zu der Längsachse angeordnet sind, wenn das Verbindungsstück an der distalen Spitze der Sonde fixiert ist.

2. Vorrichtung nach Anspruch 1, wobei das Verbindungsstück einen Sockel (110) umfasst, der über die distale Spitze passt.

3. Vorrichtung nach Anspruch 1, wobei die Vorsprünge Rollen umfassen, die konfiguriert sind, um die Oberfläche des Körpers zu kontaktieren und über diese zu rollen.

4. Vorrichtung nach Anspruch 1, wobei die Sonde eine Registriersonde ist, und
wobei die Vorrichtung des Weiteren umfasst:
eine Verarbeitungseinheit (26), die konfiguriert ist:
zum Empfangen von dreidimensionalen (3D) Bilddaten in Bezug zu mindestens einem Teil des Körpers des Patienten in einem zweiten Referenzrahmen;
zum Empfangen von Positionskoordinaten des Positionssensors in einem ersten Referenzrahmen, die durch ein Positionsverfolgungssystem erfasst wurden, das den ersten Referenzrahmen definiert, während die distale Spitze der Registriersonde die Orte auf der Oberfläche des Teils des Körpers des Patienten kontaktiert; und
zum Registrieren des ersten und des zweiten Referenzrahmens, indem die Positionskoordinaten mit entsprechenden Orten in den dreidimensionalen Bilddaten verglichen werden.

5. Vorrichtung nach Anspruch 4, wobei die dreidimensionalen Bilddaten Daten von einem Computertomographie- (CT)-System oder von einem Magnetresonanzbildgebungs- (MRT)-System umfassen.

6. Vorrichtung nach Anspruch 4, wobei der Teil des Körpers des Patienten einen Kopf des Patienten umfasst, und das 2D-Bild ein Gesicht des Patienten zeigt.

7. Vorrichtung nach Anspruch 4, wobei Registrieren des ersten und des zweiten Referenzrahmens Anwenden von relativer Skalierung, Rotationen und Translationen zwischen dem ersten und dem zweiten Referenzrahmen umfasst, um so eine Korrelation zwischen den Positionskoordinaten und den entsprechenden Orten in den dreidimensionalen Bilddaten zu maximieren.

8. Vorrichtung nach Anspruch 4, wobei die Verarbeitungseinheit konfiguriert ist, um die Orientierungspunktorte von einer versierten Bedienungsperson zu empfangen, welche die Registrierungssonde bedient, um die Orte auf dem 2D-Bild zu markieren.

9. Verfahren zum Registrieren von medizinischen Bildern, wobei das Verfahren umfasst:
Bereitstellen einer Sonde (16), umfassend einen Griff (18) mit einer Längsachse (106), eine distale Spitze (23), die auf der Längsachse angeordnet ist, und einen Positionssensor (22), der in dem Griff auf der Längsachse in einem vordefinierten Abstand (D) zu der distalen Spitze angeordnet ist, und konfiguriert ist, um ein Signal auszugeben, das einen Ort der Sonde angibt;
Passen einer Ausrichtungseinrichtung (100), umfassend drei Vorsprünge (114), die sich von einem Verbindungsstück der Ausrichtungseinrichtung erstrecken und konfiguriert sind, um eine Oberfläche (52) eines Körpers (14) eines Patienten an jeweiligen Punkten zu kontaktieren, die in einer Ebene senkrecht zu der Längsachse angeordnet sind, wenn das Verbindungsstück an der distalen Spitze der Sonde fixiert ist, über die distale Spitze der Sonde; und
Anwenden der Sonde beim Registrieren eines Positionsverfolgungssystems (12), das konfiguriert ist, um Positionskoordinaten des Positionssensors zu erfassen, mit einem Referenzrahmen von dreidimensionalen (3D) Bilddaten in Bezug auf mindestens einen Teil des Körpers des Patienten.

10. Verfahren nach Anspruch 9, wobei die Ausrichtungseinrichtung entfernbar über die distale Spitze gepasst wird.

11. Verfahren nach Anspruch 9, wobei die Vorsprünge Rollen umfassen, die konfiguriert sind, um die Oberfläche des Körpers zu kontaktieren und über diese zu rollen.

12. Vorrichtung nach Anspruch 4 oder Verfahren nach Anspruch 9, wobei das Positionsverfolgungssystem ein magnetisches Positionsverfolgungssystem umfasst, und wobei gegebenenfalls die Vorrichtung des Weiteren das Positionsverfolgungssystem umfasst.

13. Verfahren nach Anspruch 11, wobei die 3D-Bilddaten Computertomographie- (CT)-Daten oder Magnetresonanzbildgebungs- (MRT)-Daten umfassen.

14. Verfahren nach Anspruch 9, wobei der Teil des Körpers des Patienten einen Kopf des Patienten umfasst, und Anwenden der Sonde umfasst, dass die Ausrichtungseinrichtung in Kontakt mit einem Gesicht des Patienten gebracht wird.

15. Verfahren nach Anspruch 9, wobei Registrieren des Positionsverfolgungssystems Anwenden von relativer Skalierung, Rotationen und Translationen zwischen den Positionskoordinaten und dem Referenzrahmen umfasst, um so eine Korrelation zwischen den Positionskoordinaten und entsprechenden Orten in den 3D-Bilddaten zu maximieren.

## Revendications

1. Appareil médical comprenant :
une sonde (16) comprenant :
une poignée (18) ayant un axe longitudinal (106) ;
une pointe distale (23) disposée sur l'axe longitudinal ; et
un capteur de position (22), qui est disposé dans la poignée sur l'axe longitudinal à une distance prédéfinie (D) de la pointe distale et configuré pour émettre un signal indiquant l'emplacement de la sonde ; et
un gabarit d'alignement (100), qui comprend :
un connecteur (111) configuré pour être fixé de manière amovible à la pointe distale de la sonde ; et
trois protubérances (114), qui s'étendent à partir du connecteur et sont configurées pour entrer en contact avec une surface (52) du corps d'un patient (14) en des points respectifs, qui sont disposés dans un plan perpendiculaire à l'axe longitudinal lorsque le connecteur est fixé à la pointe distale de la sonde.

2. Appareil selon la revendication 1, le connecteur comprenant une douille (110), qui s'adapte sur la pointe distale.

3. Appareil selon la revendication 1, les protubérances comprenant des rouleaux, qui sont configurés pour entrer en contact et rouler sur la surface du corps.

4. Appareil selon la revendication 1, la sonde étant une sonde d'enregistrement, et
l'appareil comprenant en outre :
une unité de traitement (26) configurée :
pour recevoir des données d'images tridimensionnelles (3D) concernant au moins une partie du corps du patient dans un second cadre de référence ;
pour recevoir des coordonnées de position du capteur de position dans un premier cadre de référence acquis par un système de suivi de position, qui définit le premier cadre de référence, alors que la pointe distale de la sonde d'enregistrement entre en contact avec les emplacements sur la surface de la partie du corps du patient ; et
pour enregistrer les premier et second cadres de référence en comparant les coordonnées de position aux emplacements correspondants dans les données d'image tridimensionnelles.

5. Appareil selon la revendication 4, les données d'image tridimensionnelle comprenant des données provenant d'un système de tomographie informatisée (CT), ou d'un système d'imagerie par résonance magnétique (IRM) .

6. Appareil selon la revendication 4, la partie du corps du patient comprenant une tête du patient, et l'image 2D montrant un visage du patient.

7. Appareil selon la revendication 4, l'enregistrement des premier et second cadres de référence comprenant l'application d'une mise à l'échelle relative, de rotations et de translations entre les premier et second cadres de référence de manière à maximiser une corrélation entre les coordonnées de position et les emplacements correspondants dans les données d'image tridimensionnelle.

8. Appareil selon la revendication 4, l'unité de traitement étant configurée pour recevoir les emplacements des points de repère d'un praticien utilisant la sonde d'enregistrement pour marquer les emplacements sur l'image 2D.

9. Procédé d'enregistrement d'images médicales, le procédé comprenant :
la fourniture d'une sonde (16) comprenant une poignée (18) ayant un axe longitudinal (106), une pointe distale (23) disposée sur l'axe longitudinal, et un capteur de position (22), qui est disposé dans la poignée sur l'axe longitudinal à une distance prédéfinie (D) de la pointe distale et est configuré pour émettre un signal indiquant un emplacement de la sonde ;
l'adaptation sur la pointe distale de la sonde d'un gabarit d'alignement (100) comprenant trois protubérances (114) qui s'étendent à partir d'un connecteur du gabarit d'alignement et sont configurées pour entrer en contact avec une surface (52) du corps (14) d'un patient en des points respectifs, qui sont disposés dans un plan perpendiculaire à l'axe longitudinal lorsque le connecteur est fixé à la pointe distale de la sonde ; et
l'application de la sonde à l'enregistrement d'un système de suivi de position (12), qui est configuré pour acquérir des coordonnées de position du capteur de position, avec un cadre de référence de données d'images tridimensionnelles (3D) par rapport à au moins une partie du corps du patient.

10. Procédé selon la revendication 9, le gabarit d'alignement étant adapté de manière amovible sur la pointe distale.

11. Procédé selon la revendication 9, les protubérances comprenant des rouleaux, qui sont configurés pour entrer en contact et rouler sur la surface du corps.

12. Appareil selon la revendication 4 ou procédé selon la revendication 9, le système de suivi de position comprenant un système de suivi de position magnétique, et, éventuellement, l'appareil comprenant en outre le système de suivi de position.

13. Procédé selon la revendication 11, les données d'image 3D comprenant des données de tomographie informatisée (CT), ou des données d'imagerie par résonance magnétique (IRM).

14. Procédé selon la revendication 9, la partie du corps du patient comprenant une tête du patient, et l'application de la sonde comprenant la mise en contact du gabarit d'alignement avec le visage du patient.

15. Procédé selon la revendication 9, l'enregistrement du système de suivi de position comprenant l'application d'une mise à l'échelle, de rotations et de translations relatives entre les coordonnées de position et le cadre de référence de manière à maximiser une corrélation entre les coordonnées de position et des emplacements correspondants dans les données d'image 3D.
